# EUROPEAN PATENT SPECIFICATION

(11) **EP 1 499 325 B1**
(45) Date of publication and mention of the grant of the patent: **16.07.2008**
(21) Application number: 03738887.3
(22) Date of filing: 30.04.2003
(51) Int. Cl.: A61K 31/7016, A61K 33/30, A61P 17/00

(54) **COMPOSITION COMPRISING TREHALOSE AND ZINC AND USE THEREOF TO INHIBIT SKIN IRRITATION**
ZUSAMMESETZUNG ENTHALTEND TREHALOSE UND ZINK UND DEREN VERWENDUNG ZUR HEMMUNG DER HAUTREIZUNG
COMPOSITION COMPRENANT DU TREHALOSE ET DU ZINC ET SON UTILISATION POUR INHIBER L'IRRITATION DE LA PEAU

(30) Priority: 30.04.2002 US 376573 P
(43) Date of publication of application: 26.01.2005
(73) Proprietor: Hisamitsu Pharmaceutical Co., Inc., Tosu-shi, Saga 841-0017 (JP)
(72) Inventor: VEERAPANENI, Dange, San Diego, CA 92130 (US); INOUE, Kazutaka, San Diego, CA 92130 (US); YOSHINAGA, Takaaki c/o Hisamitsu Pharmaceutical Co, Tosu-shi, Saga 841-0017 (JP); HIRANO, Munehiko c/o Hisamitsu Pharmaceutical Co., Tosu-shi, Saga 841-0017 (JP); SATO, Shuji, Kawasaki-shi Kanagawa 216-0003 (JP)
(74) Representative: Atkinson, Jonathan David Mark
(86) International application number: PCT/US2003/013453
(87) International publication number: WO 2003/092703

(56) References cited:
- WO-A-84/02845
- WO-A-93/14773
- FR-A- 2 609 397
- BHANGANADA K ET AL: "THE USE OF SUPERSATURATED SUCROSE SOLUTION FOR CHRONIC SKIN ULCERS RESURRECTION OF AN OLD REMEDY" JOURNAL OF THE MEDICAL ASSOCIATION OF THAILAND, vol. 69, no. 7, 1986, pages 358-366, XP008021227 ISSN: 0125-2208
- "Vidal (69e édition)" 1993, EDITIONS DU VIDAL , PARIS * pages 117,118 *

## Description

### Technical Field and Background Art

The present invention relates to compositions for use in preventing or treating an adverse response to an inflammatory agent, or a skin sensitizing or skin-irritating agent using a formulation including a disaccharide and a metal ion.

FR2609397 discloses a composition with anti-inflammatory properties comprising a disaccharide selected from lactose, maltose, cellobiose, gentibiose, melibiose, saccharose and *trehalose*.

Bhanganada K. et al.: Journal of the Medical Association of Thailand (1986), 69(7), 358 - 366 shows a topical agent comprising *sucrose* syrup which promotes healing of dermal ulcers.

WO93/14773 teaches about a composition comprising disaccharides (*saccharose*), *zinc oxide* and vitamin E and the use thereof for the treatment of skin and mucous affections such as skin-fold dermatitis and acne.

WO84/02845 relates to an ointment comprising a *zinc salt* which functions as a skin cleaner and has been used for the treatment of eczema, impetigo, psoriasis and ulcers.

### Summary of the Invention

In a general embodiment of the present invention, there is provided a composition comprising an adverse skin reactive agent or skin sensitizing agent and an adverse skin reaction preventing, reducing or controlling agent comprising an effective amount of a disaccharide and a metal ion. In accordance with an embodiment of the present invention, the skin-sensitizing agent may be a therapeutic agent.

Another embodiment of the present invention the skin sensitizing agent of the composition maybe a therapeutic agent including an anti-inflammatory agent.

In yet another embodiment of the present invention, the skin sensitizing agent of the claimed composition may be an anti-inflammatory agent including methyl salicylate, acetylsalicylic acid, sodium salicylate, choline salicylate, choline magnesium salicylate, diflunisal, salflex, salicylamide, salsalate, disalcid, trolamine salicylate, trisilate, ketoprofen, prostaglandin, flurbiprofen, diclofenac, indomethacin, piroxicam, and ibuprofen.

Another embodiment of the present invention provides a composition comprising an adverse skin reactive agent or skin sensitizing agent and an adverse skin reaction preventing, reducing or controlling agent comprising an effective amount of a disaccharide and a metal ion, wherein the disaccharide is trehalose.

Yet another embodiment provides a composition comprising an adverse skin reactive agent or skin sensitizing agent and an adverse skin reaction preventing, reducing or controlling agent comprising an effective amount of a disaccharide and a metal ion, wherein the metal ion is a divalent metal ion, for example, zinc. More particularly, the metal ion is in the form of a metal oxide, for example zinc oxide.

Another embodiment of the present invention provides a use of the above compositions for the manufacture of a medicament for the treatment of an adverse skin reaction of the skin in a subject to the presence of at least one of a skin-sensitizing or a skin-irritating agent comprising the steps: providing an adverse skin reaction preventing, reducing or controlling agent comprising a disaccharide and a metal ion in a formulation; and topically administering an effective amount of the formulation to the subject so as to prevent, reduce or control the adverse skin reaction.

The skin-sensitizing agent is selected from the group consisting of therapeutic agents. and the adverse skin reaction preventing, reducing or controlling agent is administered transdermally; or, the skin-sensitizing agent is a therapeutic agent and the method further comprising administering the adverse skin reaction preventing, reducing or controlling agent and the therapeutic agent from a transdermal patch.

Yet another embodiment in accordance with the present invention provides the use of the above compositions for the manufacture of a medicament for treating an inflammatory response in a subject to the presence of at least one inflammatory agent comprising the steps: providing an inflammatory preventing, reducing or controlling agent comprising a disaccharide and a metal ion in a formulation; and administering an effective dose of the formulation to the subject, so as to prevent, reduce or control the inflammatory response.

Still another embodiment directed to preventing an inflammatory response in a subject according to the third general embodiment, further comprises administering the effective dose via topical administration including transdermal patch or tape formulation, subcutaneous administration, parentereal administration, intravenous administration, instramuscular administration, and oral administration.

Another embodiment is directed to preventing an inflammatory response according to the third general embodiment, wherein the inflammatory response is evidenced by a stimulation of cytokines, growth factors, and chemokines, for example, by a stimulation of interleukin-1α and tumor necrosis factor-α.

Yet another embodiment relates to the treatment of an inflammatory response comprising the steps: providing an inflammatory preventing, reducing or controlling agent comprising a disaccharide and a metal ion in a formulation; and topically administering an effective amount of the formulation to the subject so as to prevent, reduce or control the adverse skin reaction.

In particular the invention relates to the treatment of an inflammatory response wherein the inflammatory response is a response to the presence of an agent selected from the group consisting of methyl salicylate, acetylsalicylic acid, sodium salicylate, choline salicylate, choline magnesium salicylate, diflunisal, salflex, salicylamide, salsalate, disalcid, trolamine salicylate, and trisilate.

Still another embodiment provides the above compositions for use in treating an adverse reaction of the skin in a subject to the presence of at least one of a skin-sensitizing or a skin-irritating agent comprising the steps: providing an adverse skin reaction preventing, reducing or controlling agent comprising a disaccharide in a formulation; and administering an effective amount of the disaccharide formulation to the adverse reactive site of the subject in a suitable carrier so as to prevent, reduce or control the adverse skin reaction. More particularly, said steps may further comprise administering the effective dose of the disaccharide formulation via topical administration including transdermal patch or tape formulation, subcutaneous administration, parentereal administration, intravenous administration, instramuscular administration, and oral administration.

Another embodiment provides the above compositions for use in treating an adverse reaction of the skin in a subject to the presence of at least one of a skin-sensitizing or a skin-irritating agent comprising the steps: providing an adverse skin reaction preventing, reducing or controlling agent comprising a metal ion in a formulation; and administering an effective amount of the metal ion formulation to the adverse reactive site of the subject in a suitable carrier so as to prevent, reduce or control the adverse skin reaction. More particularly, said steps may further comprise administering the effective dose of the metal ion formulation via topical administration including transdermal patch or tape formulation, subcutaneous administration, parentereal administration, intravenous administration, instramuscular administration, and oral administration.

Yet another embodiment provides the above compositions for use in treating an inflammatory condition in a subject to the presence of at least one inflammatory agent comprising the steps: providing an inflammatory preventing, reducing or controlling agent comprising a disaccharide in a formulation; and administering an effective amount of the disaccharide formulation to the subject in a suitable carrier so as to prevent, reduce or control the inflammation. More particularly, said steps may further comprise administering the effective dose of the disaccharide formulation via topical administration including transdermal patch or tape formulation, subcutaneous administration, parentereal administration, intravenous administration, instramuscular administration, and oral administration.

Still another embodiment includes a composition effective in treating an adverse skin reaction comprising an adverse skin-reaction preventing, reducing or controlling agent comprising a therapeutically effective amount of a disaccharide and a metal ion.

Alternatively, there is provided a composition effective in treating an inflammatory response comprising an inflammatory preventing, reducing or controlling agent comprising a therapeutically effective amount of a disaccharide and a metal ion.

### Brief Description of the Drawings

The foregoing features of the invention will be more readily understood by reference to the following detailed description, taken with reference to the accompanying drawings, in which:
Fig. 1 shows the structures of Trehalose A and Trehalose B.
Fig. 2 is a graph showing the effects of Trehalose A and B on IL-1α.
Fig. 3 is a graph showing the effects of Trehalose C on IL-1α.
Fig. 4 is a graph showing the effects of Zinc Oxide on IL-1α.
Fig. 5 is a graph showing the concentration effects of Zinc Oxide on IL-1α.
Fig. 6 is a graph showing the effects of Trehalose B with Zinc Oxide on IL-1α.
Fig. 7 is a graph showing the effects of Zinc Oxide on Keratinocyte cell viability.

### Detailed Description of Specific Embodiments

Definitions. As used in this description and the accompanying claims, the following terms shall have the meanings indicated, unless the context otherwise requires:
"Inflammatory preventing agent" as used herein, means, any agent, molecule, compound, formulation, etc. capable of preventing an inflammatory response to an inflammatory agent, as indicated by repeated post-exposure measurement of non-elevated levels of cytokines, growth factors, and chemokines, particularly interleukin-1α and tumor necrosis factor-α chemokines, observed after administration of an inflammatory preventing agent with an inflammatory agent, as compared to the levels of cytokines, growth factors, and chemokines measured after administration of no inflammatory agent or measured after administration of the inflammatory agent alone.
"Inflammatory reducing agent" as used herein, means, any agent, molecule, compound, formulation, etc. capable of reducing an inflammatory response to an inflammatory agent, as indicated by post-exposure measurement of reduced levels of cytokines, growth factors, and chemokines, particularly interleukin-1α and tumor necrosis factor-α chemokines, observed after administration of an inflammatory reducing agent with an inflammatory agent, as compared to the levels of cytokines, growth factors, and chemokines measured after administration of the inflammatory agent alone.
"Inflammatory controlling agent" as used herein, means, any agent, molecule, compound, formulation, etc. capable of controlling an inflammatory response to an inflammatory agent, as indicated by repeated post-exposure measurement of unchanged levels of cytokines, growth factors, and chemokines over time, particularly interleukin-1α and tumor necrosis factor-α chemokines, observed after administration of an inflammatory controlling agent with an inflammatory agent, as compared to the levels of cytokines, growth factors, and chemokines measured over time after administration of the inflammatory agent alone.
"Adverse skin reaction preventing agent as used herein, means, any agent, molecule, compound, formulation, etc. capable of preventing an adverse skin reaction to a skin-sensitizing agent or skin-irritating agent, as indicated by objective and subjective evidence of non-itchy, non-irritated, non-painful, non-swollen, non-red, non-blotchy, non-inflamed, or otherwise normal looking and feeling skin observed and/or reported after administration of an adverse skin reaction preventing agent with a skin-sensitizing or skin-irritating agent, as compared to the objective and subjective evidence regarding skin condition observed/reported after administration of no skin-sensitizing or skin-irritating agent or measured after administration of the skin-sensitizing or skin-irritating agent alone.
"Adverse skin reaction reducing agent" as used herein, means, any agent, molecule, compound, formulation, etc. capable of reducing an adverse skin reaction to a skin-sensitizing or skin-irritating agent, as indicated by objective and subjective evidence of non-itchy, non-irritated, non-painful, non-swollen, non-red, non-blotchy, non-inflamed, or otherwise normal looking and feeling skin observed and/or reported after administration of an adverse skin reaction preventing agent with a skin-sensitizing or skin-irritating agent, as compared to the objective and subjective evidence regarding skin condition observed/reported after administration of the skin-sensitizing or skin-irritating agent alone.
"Adverse skin reaction controlling agent" as used herein, means, any agent, molecule, compound, formulation, etc. capable of controlling an adverse skin reaction to a skin-sensitizing or skin-irritating agent, as indicated by sustained objective and subjective evidence of non-itchy, non-irritated, non-painful, non-swollen, non-red, non-blotchy, non-inflamed, or otherwise normal looking and feeling skin observed and/or reported after administration of an adverse skin reaction controlling agent with a skin-sensitizing or skin-irritating agent, as compared to the sustained objective and subjective evidence regarding skin condition observed/reported after administration of the skin-sensitizing or skin-irritating agent alone.

Epidermal Keratinocytes play an active role in the irritation of primary contact-irritancy and contact-hypersensitivity reaction in the skin through the synthesis and production of proinflammatory mediatory like cytokines, growth factors and chemokines, including IL-1α, and TNF-α. Methyl Salicylate (MS) is an active ingredient in oil of wintergreen, which is used, in various over-the-counter topical preparations indicated to relieve musculo-skeletal pains and aches. The potential systemic toxicity and the risk of skin irritation of the topical preparations is well documented in the literature and has been attributed to diverse actions of the compounds like MS on human cells.

Previously, we have shown that MS stimulates release of the primary cytokines, especially IL-1α, in epidermal keratinocytes and anti-irritants like ethacrynic acid (ETA) inhibited the IL-1α release by MS. These results suggested that cytokine release by epidermal keratinocytes could be used as a marker for predicting the skin irritation potential of topically applied MS. The current study examined the effectiveness of commercially available trehalose, a disaccharide, to block the MS stimulated IL-1α release. We used three commercially available trehalose preparations: namely, trehalose A, B, and C. The three trehalose preparations used in this study differed in that one contained trace amounts of metal ions (trehalose A) and the other two were highly purified forms (trehalose B and trehalose C) which do not contain any metal ions. Results showed that only trehalose A was capable of inhibiting MS induced IL-1α release compared to trehalose B and C, which had no effect. Chemical analysis of these trehalose preparations confirmed that the only difference between the three preparations was the presence of metal ions in the trehalose A preparation. The analysis also indicated that zinc was the most abundant metal ion found in the trehalose A preparation. Hence, the ability of zinc oxide to inhibit MS induced IL-1α was tested. Results showed that ZnO was able to block MS induced IL-1α production as effectively as trehalose A, thus revealing a novel anti-irritant effect of these compounds in epidermal keratinocytes. These findings suggest that trehalose A or zinc oxide could be used in transdermal drug delivery systems to prevent skin irritation.

### Methods:

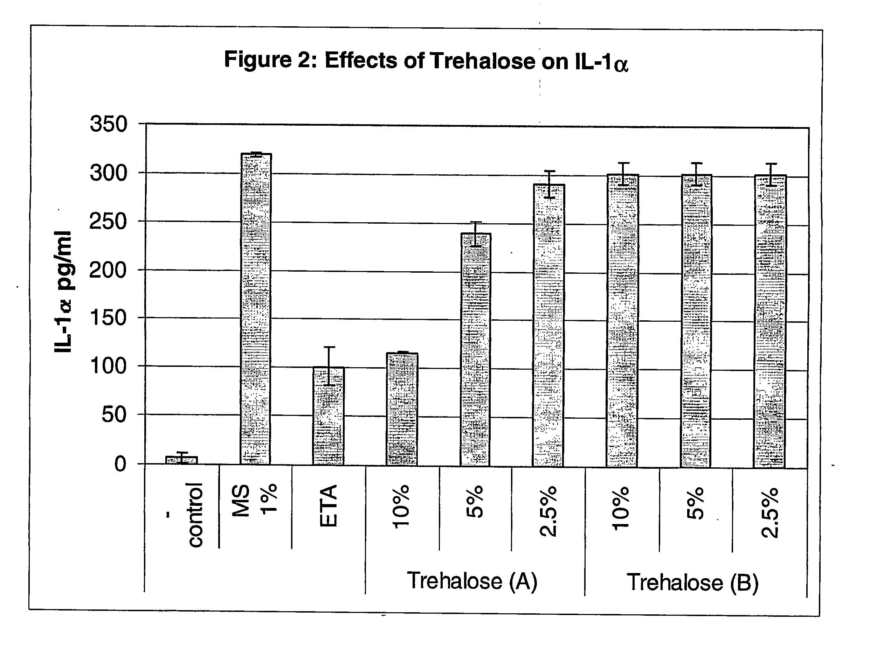

Reagents methyl salicylate, ethacrynic acid, trehalose A, B, and C were obtained from Sigma-Aldrich (St. Louis, MO). Trehaloses were dissolved in keratinocyte media. Methyl salicylate was diluted in keratinocyte media. Ethacrynic acid was dissolved in DMSO. Keratinocyte cells were grown to 100% confluency as per the instructions given by ATCC. Then the cells were trypsinized to dislodge cells and spun at 3000 rpm for 3 min. The supernatant was aspirated and the cells were re-suspended in fresh media at a density of 1.0X10⁶ cells per mL. The cell suspension was the pipetted into a 96-well plate (200ul of per well) and cells were incubated overnight at 37°C. The next day morning media was aspirated, and 100 uL of fresh medium containing methyl salicylate (2%) was added to all the wells except for the (-) control wells. No MS was included in the (-) control wells. Then 100 uL of medium containing the various test reagents were added to the wells so that the final concentrations were as indicated in figures 2-7. The 96-well plate was then incubated. After incubation for four hours at 36°C, the supernatant from the wells was transferred to centrifuge tubes and centrifuged to remove the cell debris. Then the presence of IL-1α was determined using ELISA kit (R&D systems, Inc.) as per the manufacturer's specifications. The cells remaining in the wells were treated with pre-warmed media containing MTT solution and incubated for four hours. Then the formazan product (metabolic product of MTT) in the wells was solubilized and the absorbance was measured using a plate reader.

### Results:

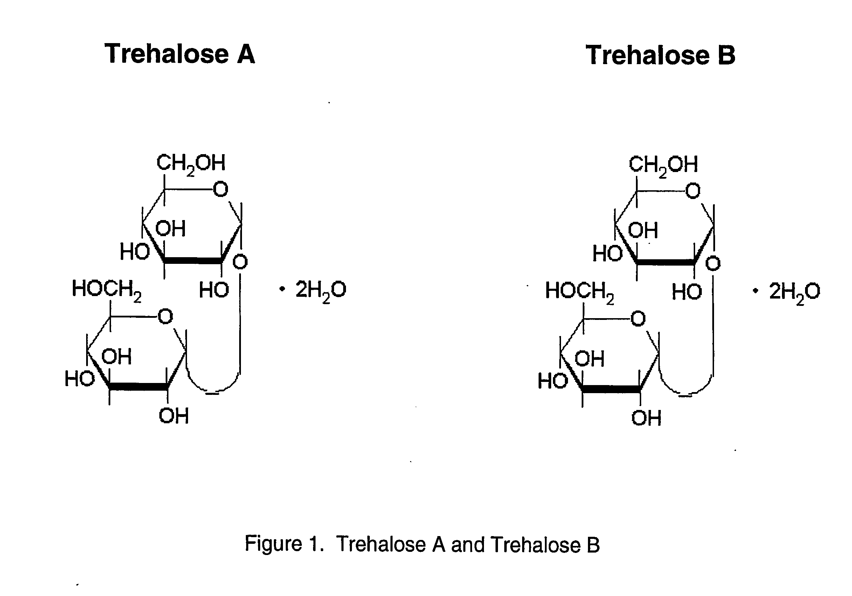

The three different varieties of trehalose preparations used in this study (trehalose A, B, and C) had the same chemical formula and structure as well as the same optical isomerism (see figure 1). The only difference between the preparations is the manner in which they were purified. The purification process removed metal ions from trehalose B and trehalose C preparations, whereas the trehalose A preparation showed trace amounts of zinc ions.

Human keratinocytes were stimulated with MS in the absence or the presence of trehalose compounds, to find an optimal concentration of trehalose at which IL-1α production is reduced by half compared to the positive control.

Figure 2 shows the effects of higher concentrations of Trehalose A and B on IL-1alpha concentrations. Ethacrynic Acid (ETA) at 0.16%, and 10% Trehalose A exhibit over 50% reduction in IL-1a levels compared to MS 1% (positive control). 5% Trehalose A has much lower levels of reduction while 2.5% Trehalose A has no effect. At 10% Trehalose A the reduction in IL-1a is almost as great as the reduction of 0.16% Ethacrynic acid. Trehalose B at any of these concentrations has no effect. Higher concentrations than 10% of Trehalose A, B or C were not chosen because of the difficulty involved in dissolving the samples. At low concentrations of 0.1 - 2.5%, neither trehalose A nor B showed any effect.

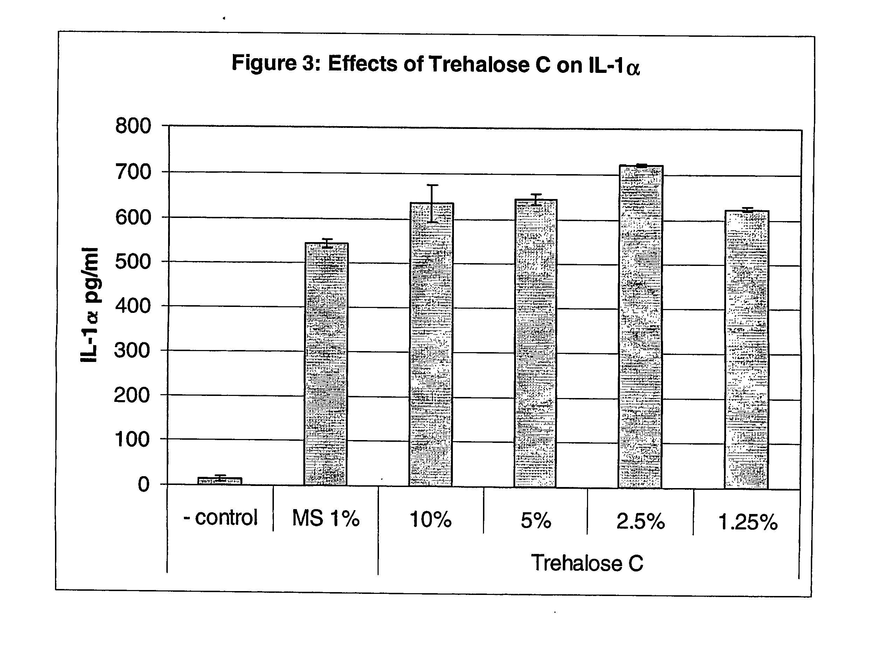

Figure 3 shows the effects of Trehalose C on IL-1a levels in Keratinocyte cells stimulated by 1% Methyl Salicylate. In contrast, trehalose B and C had no effect at any concentrations that were tested (see figures 2 and 3). Since the inhibition of IL-1α occurred only with trehalose A and the difference between the trehaloses tested was only the presence of zinc in trehalose A, experiments were performed to determine whether the inhibitory effect of trehalose A was caused by zinc or the trehalose A compound alone.

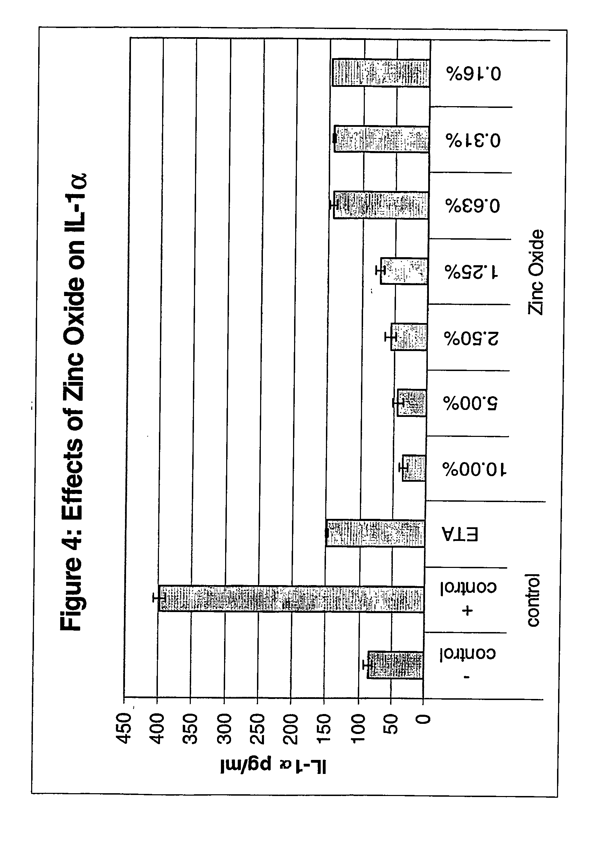

Experiments were performed using zinc oxide alone, or in combination with trehalose A, B, or C. Figure 4 shows the effects of high concentrations of Sample A (Zinc Oxide alone) on IL-1alpha levels in Keratinocyte cells stimulated with 1% Methyl Salicylate. The - control, + control and Ethacrynic acid (0.16%) wells are as previously shown in Figs. 1-3. When we incubated the cells with high concentrations of zinc oxide, there was a dramatic reduction of MS stimulated IL-1α levels, but the cells were not active. Even at high to medium concentrations the IL-1α levels were significantly reduced indicating possible anti-irritancy potential of Zinc Oxide. Sample A shows a large decrease in IL-1α levels at the concentration levels 10% - 1.25%. At concentrations of 0.63% and below Sample A shows an inhibition of IL-1α similar to the inhibition shown by ETA (Ethacrynic Acid 0.16%). The main difference between Trehalose A and B is the presence of the metal ion zinc, which has been removed from Trehalose B preparations.

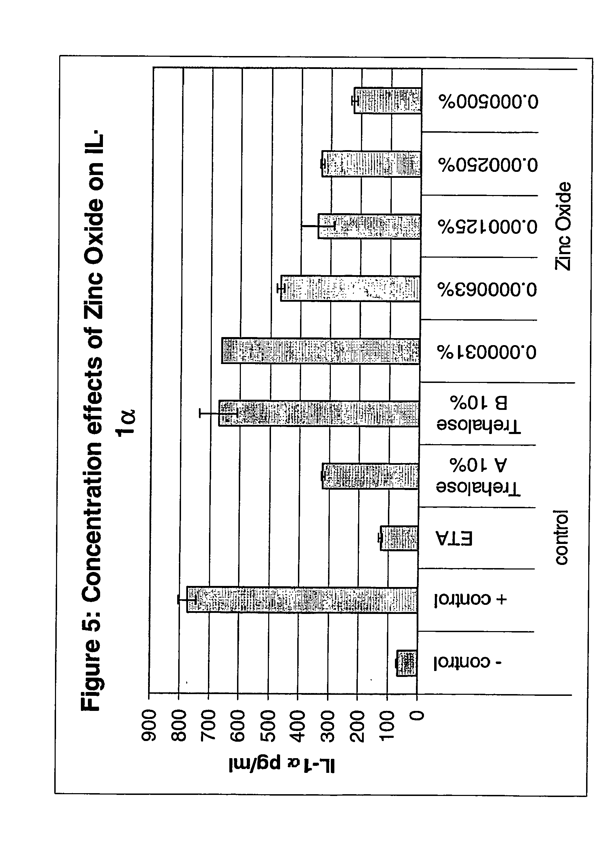

Further studies showed that even at lower concentrations zinc oxide showed a dose dependent decrease in methyl salicylate induced IL-1α levels in keratinocyte cells. Figure 5 shows the effects of ETA (Ethacrynic acid), Trehalose A and B, and Sample A (Zinc Oxide) on IL-1α levels in Keratinocyte cells stimulated by 1% Methyl Salicylate. The - control, + control, and ETA (Ethacrynic acid 0.16%) wells show IL-1α concentrations as previously shown. Trehalose A 10% shows greater than 50% inhibition of IL-1alpha concentration. Trehalose B has no effect on IL-1α levels in Keratinocyte cells stimulated with 1% Methyl Salicylate. Sample A (Zinc Oxide) shows a significant dose dependent decrease in IL-1α concentrations. At very low levels of Zinc Oxide concentration the reduction of IL-1α in Keratinocytes induced by Methyl Salicylate is shown in a dose dependent manner. At concentrations as low as .000125% Zinc Oxide clearly shows a greater than 50% reduction in IL-1α concentrations indicating possible anti-irritancy potential.

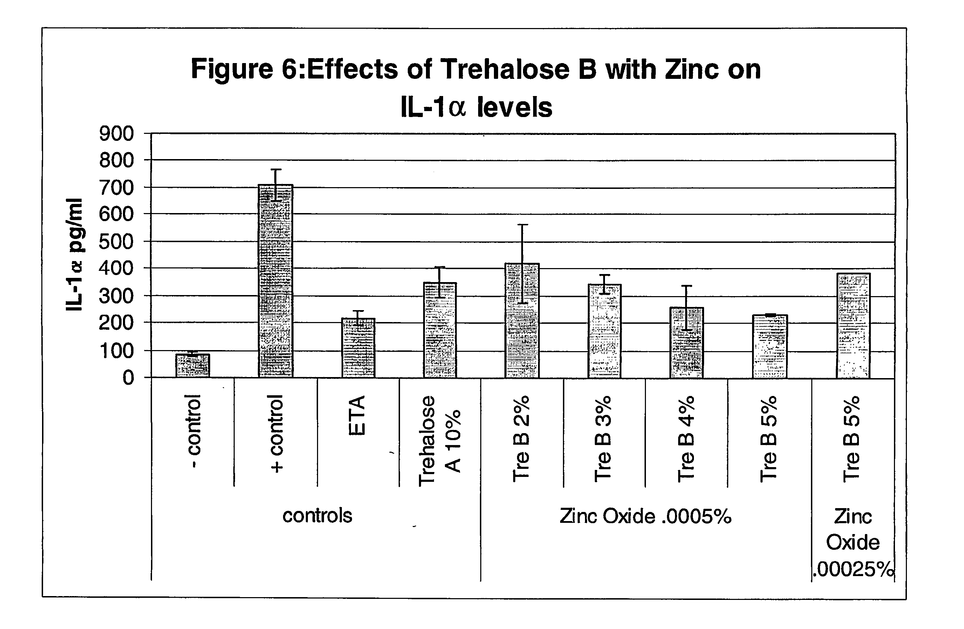

These results suggest that the inhibition was dependent on the presence of zinc in trehalose A. In order to confirm these results, we used a combination of zinc oxide and trehalose B (which is inactive). Figure 6 shows the effects of Trehalose B combined with Zinc Oxide on IL-1a levels in Keratinocyte cells. The controls for this experiment show data that is consistent with previous experiments. Zinc Oxide in combination with Trehalose B shows a large dose dependent decrease in IL-1α concentrations induced by Methyl Salicylate. As Zinc Oxide levels are decreased there is an increase in IL-1alpha concentrations at the same Trehalose B concentration indicating that Trehalose B along with Zinc lower IL-1α concentrations. Thus, interestingly, trehalose B in combination with zinc oxide was able to inhibit MS stimulated IL-1α levels.

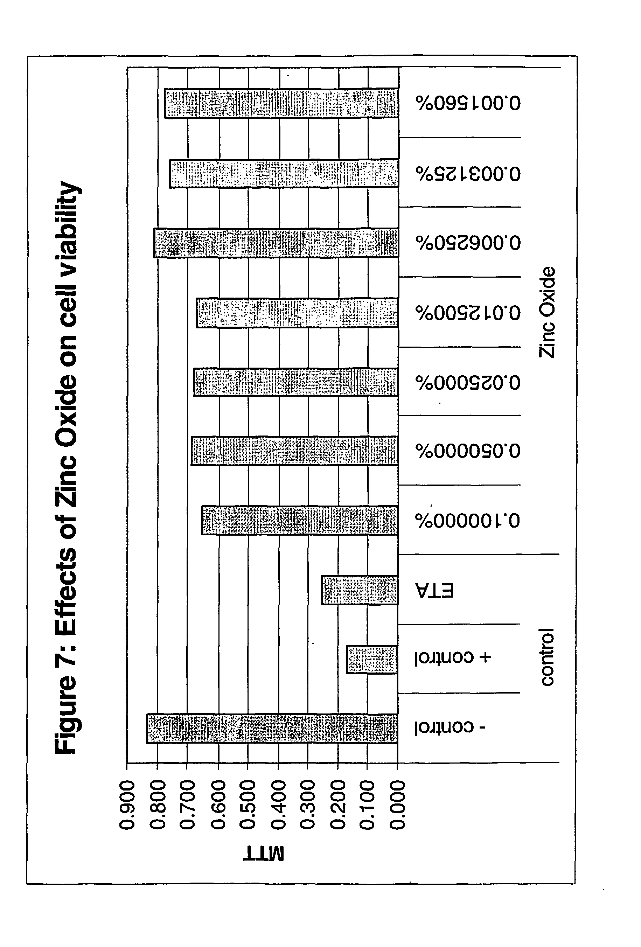

We also tested the cytotoxicity of zinc oxide on keratinocyte cells using the MTT assay as described in the Methods section. Figure 7 shows the effects of Zinc Oxide on MTT levels in Keratinocyte cells. The - control, + control and ETA (Ethacrynic acid 0.16%) wells are as previously shown. Zinc Oxide shows no effect on MTT concentration levels in Keratinocyte cells, as indicated by healthy cells metabolizing MTT into the purple dye formazan salt. Thus, the concentrations at which ZnO inhibited the MS stimulated IL-1α release had no effect on cell viability.

## Claims

1. A composition comprising:
an anti-inflammatory agent selected from the group consisting of methyl salicylate, acetylsalicylic acid, sodium salicylate, choline salicylate, choline magnesium salicylate, diflunisal, salflex, salicylamide, salsalate, disalcid, trolamine salicylate, trisilate, ketoprofen, prostaglandin, flurbiprofen, diclofenac, indomethacin, piroxicam, and ibuprofen; and
a therapeutically effective amount of a disaccharide and a metal ion, in which the disaccharide is trehalose and the metal ion is zinc.

2. A composition according to claim 1, wherein the zinc ion is in the form of zinc oxide.

3. A composition according to claim 2, wherein the anti-inflammatory agent is methyl salicylate.

4. Use of a composition as claimed in any of claims 1 to 3 in the preparation of a medicament for the topical treatment of an adverse skin reaction of the skin in a subject in the presence of at least one of a skin-sensitizing or a skin-irritating agent.

5. Use according to claim 4 wherein the composition is administered transdermally.

6. Use according to claim 5, wherein the skin-sensitizing agent is methyl salicylate or acetylsalicylic acid or salts thereof and the medicament is in the form of a transdermal patch.

7. Use of a composition as claimed in any of claims 1 to 3 in the preparation of a medicament for the treatment of an inflammatory response in a subject in the presence of at least one inflammatory agent.

8. Use according to claim 7, further comprising administering the effective dose via topical administration including transdermal patch or tape formulation, subcutaneous administration, parentereal administration, intravenous administration, instramuscular administration, and oral administration.

9. Use according to claim 7, wherein treatment of the inflammatory response is evidenced by a reduction in the stimulation of cytokines, growth factors, and chemokines relative to the stimulation of cytokines, growth factors and chemokines observed in the absence of administration of an inflammatory preventing, reducing or controlling agent.

10. Use according to claim 9, wherein treatment of the inflammatory response is evidenced by a reduction in the stimulation of interleukin-1α and tumor necrosis factor-α chemokines relative to the stimulation of cytokines, growth factors and chemokines observed in the absence of administration of an inflammatory preventing, reducing or controlling agent.

11. Use according to claim 4, wherein the composition is administered by:
transdermal patch or tape formulation, subcutaneous administration, parentereal administration, intravenous administration, instramuscular administration, or oral administration.

12. Use according to claim 4, wherein the skin-sensitizing or skin-irritating agent is the anti-inflammatory agent methyl salicylate, acetylsalicylic acid, or a combination thereof.

## Patentansprüche

1. Zusammensetzung, umfassend:
ein antiinflammatorisches Mittel, ausgewählt aus der Gruppe, bestehend aus Methyl, Salicylat, Acetylsalicylsäure, Natriumsalicylat, Cholinsalicylat, Cholinmagnesiumsalicylat, Diflunisal, Salflex, Salicylamid, Salsalat, Disalcid, Trolaminsalicylat, Trisilat, Ketoprofen, Prostaglandin, Flurbiprofen, Diclofenac, Indomethacin, Piroxicam und Ibuprofen, und
eine therapeutisch wirksame Menge eines Disaccharids und eines Metallions, wobei das Disaccharid Trehalose ist und das Metallion Zink ist.

2. Zusammensetzung nach Anspruch 1, wobei das Zinkion in der Form von Zinkoxid ist.

3. Zusammensetzung nach Anspruch 2, wobei das antiinflammatorische Mittel Methylsalicylat ist.

4. Verwendung einer Zusammensetzung, wie sie in einem der Ansprüche 1 bis 3 beansprucht ist, bei der Herstellung eines Medikaments für die topische Behandlung einer ungünstigen Hautreaktion der Haut bei einem Subjekt in Gegenwart wenigstens eines von einem hautsensibilisierenden oder einem hautirritierenden Mittel.

5. Verwendung nach Anspruch 4, wobei die Zusammensetzung transdermal verabreicht wird.

6. Verwendung nach Anspruch 5, wobei das hautsensibilisierende Mittel Methylsalicylat oder Acetylsalicylsäure oder Salze davon ist und das Medikament in der Form eines transdermalen Pflasters ist.

7. Verwendung einer Zusammensetzung, wie sie in einem der Ansprüche 1 bis 3 beansprucht ist, in der Herstellung eines Medikaments für die Behandlung einer inflammatorischen Reaktion bei einem Subjekt in Gegenwart wenigstens eines inflammatorischen Mittels.

8. Verwendung nach Anspruch 7, außerdem umfassend: Verabreichen der wirksamen Dosis über eine topische Verabreichung, einschließlich transdermales Pflaster oder Tape-Formulierung, subkutane Verabreichung, parenterale Verabreichung, intravenöse Verabreichung, intramuskuläre Verabreichung und orale Verabreichung.

9. Verwendung nach Anspruch 7, wobei eine Behandlung der inflammatorischen Reaktion bewiesen wird durch eine Reduktion bei der Stimulation von Cytokinen, Wachstumsfaktoren und Chemokinen, verglichen mit der Stimulation von Cytokinen, Wachstumsfaktoren und Chemokinen, die in Abwesenheit einer Verabreichung eines eine Entzündung verhindernden, reduzierenden oder kontrollierenden Mittels beobachtet wird.

10. Verwendung nach Anspruch 9, wobei eine Behandlung der inflammatorischen Reaktion bewiesen wird durch eine Reduktion bei der Stimulation von Interleukin-1α- und Tumornekrosefaktor-α-Chemokinen im Vergleich zu der Stimulation von Cytokinen, Wachstumsfaktoren und Chemokinen, die in Abwesenheit einer Verabreichung eines eine Entzündung verhindernden, reduzierenden oder kontrollierenden Mittels beobachtet wird.

11. Verwendung nach Anspruch 4, wobei die Zusammensetzung verabreicht wird durch: transdermales Pflaster oder Tape-Formulierung, subkutane Verabreichung, parenterale Verabreichung, intravenöse Verabreichung, intramuskuläre Verabreichung oder orale Verabreichung.

12. Verwendung nach Anspruch 4, wobei das hautsensibilisierende oder hautirritierende Mittel das antiinflammatorische Mittel Methylsalicylat, Acetylsalicylsäure oder eine Kombination davon ist.

## Revendications

1. Composition comprenant :
un agent anti-inflammatoire choisi dans le groupe constitué par le salicylate de méthyle, l'acide acétylsalicylique, le salicylate de sodium, le salicylate de choline, le salicylate de choline et de magnésium, le diflunisal, le salflex, le salicylamide, le salsalate, le disalcide, le salicylate de trolamine, le trisilate, le cétoprofène, la prostaglandine, le flurbiprofène, le diclofénac, l'indométhacine, le piroxicam et l'ibuprofène ; et
une quantité thérapeutiquement efficace d'un disaccharide et d'un ion métal, dans laquelle ledit saccharide est le tréhalose et l'ion métal est le zinc.

2. Composition selon la revendication 1, dans laquelle l'ion zinc est sous la forme d'oxyde de zinc.

3. Composition selon la revendication 2, dans laquelle l'agent anti-inflammatoire est le salicylate de méthyle.

4. Utilisation d'une composition selon l'une quelconque des revendications 1 à 3 dans la préparation d'un médicament pour le traitement topique d'un effet cutané indésirable de la peau chez un sujet en présence d'au moins l'un d'un agent allergène cutané ou d'un agent d'irritation de la peau.

5. Utilisation selon la revendication 4, dans laquelle la composition est administrée par voie transdermique.

6. Utilisation selon la revendication 5, dans laquelle l'agent allergène cutané est le salicylate de méthyle ou l'acide acétylsalicylique ou des sels de ceux-ci et le médicament est sous la forme d'un timbre transdermique.

7. Utilisation d'une composition selon l'une quelconque des revendications 1 à 3, dans la préparation d'un médicament pour le traitement d'une réaction inflammatoire chez un sujet en présence d'au moins un agent inflammatoire.

8. Utilisation selon la revendication 7, comprenant en outre l'administration de la dose efficace via administration topique comprenant une formulation de bande ou de timbre transdermique, une administration sous-cutanée, une administration parentérale, une administration intraveineuse, une administration intramusculaire et une administration orale.

9. Utilisation selon la revendication 7, dans laquelle le traitement de la réaction inflammatoire est montré par une réduction dans la stimulation de cytokines, de facteurs de croissance et de chimiokines relatives à la stimulation de cytokines, de facteurs de croissance et de chimiokines observée en l'absence d'administration d'un agent de prévention, de réduction ou de régulation inflammatoire.

10. Utilisation selon la revendication 9, dans laquelle le traitement de la réaction inflammatoire est montré par une réduction de la stimulation d'interleukine-1α et de chimiokines de facteur onconécrosant-α relative à la stimulation de cytokines, de facteurs de croissance et de chimiokines observée en l'absence d'administration d'un agent de prévention, de réduction ou de régulation inflammatoire.

11. Utilisation selon la revendication 4, dans laquelle la composition est administrée par: formulation de bande ou de timbre transdermique, administration sous-cutanée, administration parentérale, administration intraveineuse, administration intramusculaire ou administration orale.

12. Utilisation selon la revendication 4, dans laquelle l'agent allergène cutané ou d'irritation de la peau est l'agent anti-inflammatoire salicylate de méthyle, acide acétylsalicylique ou une combinaison de ceux-ci.
